# EUROPÄISCHE PATENTSCHRIFT

(11) **EP 1 275 397 B1**
(45) Veröffentlichungstag und Bekanntmachung des Hinweises auf die Patenterteilung: **06.08.2014**
(21) Anmeldenummer: 02015881.2
(22) Anmeldetag: 19.05.2000
(51) Int. Cl.: A61K 38/19, A61K 38/39, A61K 35/12, A61K 45/06, A61K 38/36, A61K 38/48, A61K 38/57

(54) **Arzneimittel zur lokalen Anwendung**
Medicament for local use
Médicament pour l'emploi local

(30) Priorität: 19.05.1999 AT 89599
(43) Veröffentlichungstag der Anmeldung: 15.01.2003
(62) Teilanmeldung aus: 00936524.8
(73) Patentinhaber: Bio & Bio Licensing SA, 1143 Luxembourg (LU)
(72) Erfinder: Eibl, Johann, Dr., 1180 Wien (AT)
(74) Vertreter: Schwarz, Albin

(56) Entgegenhaltungen:
- BLOMBACK,BIRGER; BLOMBACK, MARGARETA: "Purification of human and bovine fibrinogen" ARKIV EMI, Bd. 10, 1956, Seiten 415-443, XP009000987 Karolinska Inst., Stockholm
- DATABASE WPI Section Ch, Week 199414 Derwent Publications Ltd., London, GB; Class B04, AN 1994-111920 XP002223492 & JP 06 054897 A (KAGAKU OYOBI KESSEI RYOHO), 1. März 1994 (1994-03-01)

## Beschreibung

Der Wundverschluß und die damit im Zusammenhang stehende Blutstillung erfolgt physiologisch durch Gerinnung des austretenden Blutes im Wundbett, wodurch der Verschluß von kleinen Blutgefäßen und Kapillaren zustandekommt. Die danach einsetzende Wundheilung erfolgt mit Hilfe der durch das geronnene Blut gebildeten, provisorischen extrazellulären Matrix (ECM) (Clark, R.A.F. et al., 1982, J. Invest. Dermatol. 70:264-269; Clark, R.A.F.(ed.), 1996, The Molecular and Cellular Biology of Wound Repair, Plenum Press, New York). Diese Matrix besteht, abgesehen von Blutzellen im wesentlichen aus Fibrin als Gerüstsubstanz, die als Reservoir für eine Reihe von Plasmaproteinen dient, die für die einsetzende Wundheilung von Bedeutung sind, wie Fibronektin (Mosesson, M.W. und Umfleet, R., 1970, J. Biol. Chem. 245:5726-5736; Clark, R.A.F. et al., 1982, J. Invest. Dermatol. 70:264-269), Vitronektin (Preissner, K.T. und Jenne, D., 1991, Thromb. Haemost. 66:189-194), Plasminogen (Castellino,F.J. et al., 1983, Ann. NY Acad. Sci. 408:595-601), Plasminogenaktivator (Thorsen, S. et al., 1972, Thromb. Pathol. Haemost. 28:65-74), Plasminogenaktivator-Inhibitor (Wagner, O.F. et al., 1989, Blood 70:1645-1653), und alpha₂-Plasmininhibitor (Sakata, Y. und Aoki, N., 1980, J. Clin. Invest. 65:290-297).

Die Menge von Plasminogenaktivator, Plasminogenaktivator-Inhibitor und alpha₂-Plasmininhibitor und das Verhältnis zueinander üben eine wesentliche Kontrolle auf den nachfolgenden Abbauprozeß des Fibrins aus. In diesem Fibringerüst sind aber noch andere Substanzen, wie z.B. Thrombin, TGF-beta und PDGF enthalten, die für die Einwanderung von Zellen und die mehrfache Remodellierung (Umbau) der provisorischen ECM zu der endgültigen ECM mit den entsprechenden Zellpopulationen erforderlich sind.

Als erstes wandern in größerer Menge Granulozyten in das Wundgebiet und in den Wundverschluß ein. Sie setzen verschiedene, für den einsetzenden Wundheilungsvorgang wichtige Substanzen, insbesondere Kollagenasen und Elastasen, frei und zerstören extrazellulär und intrazellulär Mikroorganismen, die ins Wundgebiet gelangt sind und die sich dort vermehren können. Während die Einwanderung von Granulozyten zu Ende geht, treten im Wundgebiet vermehrt Monozyten inklusive Makrophagen auf. Am dritten Tag kommt es zum Aussprossen von Fibroblasten im Wundgebiet, die über Fibronektinstränge bis an die Oberfläche des Wundverschlusses gelangen. Gefolgt von der Einsprossung von Blutkapillaren kommt es zu einer Reihe von Zelltransformationen und Remodellierungsprozessen, die in den meisten Fällen wieder zur vollständigen Integrität des verletzten Gewebes führen (Clark, R.A.F.(ed.), 1996, The Molecular and Cellular Biology of Wound Repair, Plenum Press, New York).

Den ersten Versuchen, die bereits 80 bis 90 Jahre zurückliegen, mit Hilfe von Blutplasma Wunden zu verschließen, waren wegen der relativ niedrigen Viskosität im Vergleich zu Blut, der geringen Adhärenz im Wundbett und wegen der hohen Fragilität eines sich allenfalls aus Plasma ausbildenden Clots von geronnenem Plasma kein Erfolg beschieden.

Die Verwendung von angereicherten Fibrinogenlösungen anstelle von Plasma zur Blutstillung und zum Wundverschluß war anfangs ebenfalls ohne Erfolg, doch schließlich konnte durch Abhebung der Fibrinogenkonzentration solcher fibrinogenhältiger Lösungen um mehr als das 10-fache des Fibrinogenspiegels im Plasma ein wesentlicher Erfolg erzielt werden (Löblich, 1975, unpublizierte Mitteilung).

Bei der Umwandlung von Fibrinogen in Fibrin besteht die Möglichkeit, daß der hämostatisch wirksame Fibrinwundverschluß sich nach einigen Stunden durch Wundbettenzyme ablöst und es dadurch zur Nachblutung kommt. Die Ablösung des Fibrinwundverschlusses vom Wundbett ist der wesentlich häufigere und daher gefährlichere Vorgang als die Fibrinolyse des gesamten Fibrinwundverschlusses.

Schon bei den ersten erfolgreichen Anwendungen von hochkonzentrierten Fibrinogenlösungen (Matras, H. et al., 1972, Wr. Med. Wschr. 122:517-523) und der Umwandlung von Fibrinogen in Fibrin durch Thrombin im Wundgebiet zeigte sich, daß die Abhebung des Fibrinwundverschlusses und die damit meist eintretenden Nachblutungen durch Fibrinolyseinhibitoren vermieden werden kann. Von den versuchten niedermolekularen Inhibitoren konnten Epsilonaminokapron-Säure und Derivate als wirksam befunden werden, allerdings wiesen sie den Nachteil auf, schnell aus dem geronnenen Fibrin und aus dem Wundgebiet zu diffundieren und somit ihre lokale Wirksamkeit zu verlieren.

Die Zumischung von hochmolekularen Inhibitoren, wie z.B. Aprotinin (Trasylol), konnte erfolgreich durchgeführt werden, indem es nur zu einer langsamen Diffusion des Inhibitors aus dem Wundgebiet kommt, allerdings mit dem Nachteil, daß es sich hierbei um einen bovinen und somit xenogenen Proteaseinhibitor handelt, der potentiell Allergien und Anaphylaxien hervorrufen kann. Neuerdings sind auch wegen der potentiellen Übertragungsfähigkeit von Zoonosen Einwände gegen die Verwendung tierischen Materials für die parenterale Anwendung beim Menschen gemacht worden.

In der WO-A - 99/11301 wird der Vorschlag gemacht, anstelle von Aprotinin Elastaseinhibitoren oder andere gegen Leukozytenproteasen wirksame Inhibitoren zu verwenden. Dieser Vorschlag bewirkt jedoch nach Erkenntnis des Erfinders der gegenständlichen Erfindung verschiedene Nachteile. Zwar sind diese Inhibitoren direkt oder indirekt durch Inhibition von Enzymen, die das fibrinolytische System aktivieren können, wirksam, sie können jedoch durch starke Hemmung der von den Granulozyten freigesetzten Proteasen, wie Kollagenasen und Elastasen, die Einleitung der Wundheilung nach Einwanderung der Granulozyten in das Wundgebiet stören.

Eine weitere Schwierigkeit bei der Herstellung von fibrinogen- und thrombinhältigen pharmazeutischen Arzneimitteln, die einen allogenen Proteaseinhibitor und ein Transglutaminasezymogen enthalten, ist durch die seit längerer Zeit erforderliche Virusinaktivierung solcher Zubereitungen gegeben. Bei der Virusinaktivierung geht meist ein großer Teil der Aktivität des in der Zubereitung enthaltenen Proteaseinhibitors bzw. Transglutaminasezymogens verloren, so daß die nach Durchführung des Virusinaktivierungsverfahrens erhaltenen Zubereitungen oft nur mehr eine geringe Aktivität des Proteaseinhibitors bzw. Transglutaminasezymogens aufweisen. Dadurch kann es zur unzureichenden Hemmung von im Wundbett vorhandenen fibrinolytischen Enzymen und in der Folge zur Ablösung des Fibrinwundverschlusses vom Wundbett kommen.

Konzentrierte Fibrinogenlösungen weisen eine Reihe von Nachteilen auf. Sie sind von einer geringen Lagerstabilität und müssen zur Lagerung tiefgefroren oder gefriergetrocknet werden und können durch Auftauen bzw. Wiederauflösen nicht immer befriedigend verwendbar gemacht bzw. rekonstituiert werden. Außerdem nimmt die Auflösung eines Fibirnogenlyophilisats geraume Zeit in Anspruch. Lösungsvermittler oder leicher lösliche Fibrinogene sind in den meisten Fällen zelltoxisch und daher für eine ungestörte Wundheilung nicht angebracht.

Die größte Schwierigkeit bei der Lagerung fibrinogenhältiger Produkte ist die Instabilität des Fibrinogens, da Spuren von Verunreinigungen mit Gerinnungsfaktoren eine langsame Umwandlung von Fibrinogen in unlösliches Fibrin bedingen, was dann die Anwendung eines solchen pharmazeutischen Präparates nicht mehr erlaubt.

Diesen Nachteil will die vorliegende Erfindung überwinden.

Die vorliegende Erfindung betrifft daher ein Verfahren zur Herstellung einer fibrinogenhältigen Lösung, die bei Kühlschranktemperatur oder Raumtemperatur lagerfähig ist, und ist dadurch gekennzeichnet, daß die Fibrinogenlösung aus gentechnologisch hergestelltem Fibrinogen oder aus Plasma gewonnenem Fibrinogen durch Fraktionierung mit Glyzin bei Temperaturen unter 0°C hergestellt wird.

Erfindungsgemäß hergestellte fibrinogenhältige Lösungen sind stabil und können im flüssigen Zustand mit oder ohne Stabilisatoren über zwei Jahre bei einer Temperatur von 4°-8°C oder eingefroren oder gefriergetrocknet gelagert werden, ohne daß dabei unlösliches Fibrin ausfällt oder sich Fibrinogenspaltprodukte durch die Entstehung von Plasmin während der Lagerung in einem Ausmaß bilden, daß die Gerinnbarkeit gestört wird. Solche Lösungen gerinnen weder nach Zusatz von Thromboplastin und Taipan Viper Venom, noch durch Zusatz von aktiviertem partiellem Thromboplastin. Bei Lagerung im tiefgefrorenen Zustand und nach dem Wiederauftauen der erfingdungsgemäß hergestellten fibrinogenhältigen Lösungen werden keine Gerinnungsvorgänge induziert, und die erhaltenen Lösungen sind zumindest einige Stunden stabil. Gefriergetrocknete Präparationen sind leicht und vollständig mit entsprechenden Lösungsmitteln rekonstituierbar.

### Beispiele

### 1. Beispiel (Erfindung) :

### Gewinnung einer stabilen Fibrinogenlösung.

100 1 Plasma geeignet zur Gewinnung von Arzneimitteln oder die daraus mit Hilfe einer Kälte Äthanol-Fällung gewonnene Cohn Fraktion-I oder durch Einfrieren des Plasmas und vorsichtiges Auftauen gewonnenes Kältepräzipitat werden als Ausgangsmaterial verwendet.

Das Plasma wird als solches, die Cohn-Fraktion-I und das Kältepräzipitat nach dem Auflösen in etwa 20 Liter 0.9%iger NaCl und 0.1%igem Natriumzitratpuffer pH7 mit festem Glyzin mit - einem Reinheitsgrad für pharmazeutische Zubereitungen bis zur Sättigung versetzt und dabei auf-2° C bis - 3 °C abgekühlt, mindestens 10 bis maximal 15 Stunden bei dieser Temperatur gelagert, von ungelöstem Glyzin abgetrennt und der fibrinögen-fibronektinhältige Niederschlag durch Zentrifugieren in einer Schnelllaufzentrifuge abgetrennt.

Die Überstände können für weitere Verarbeitung verwendet werden, um daraus noch weitere Plasmaproteine zu isolieren.

Der gallertartige Niederschlag wird aus dem Zentrifugenrotor entfernt, gelöst und wie vorher mit Glyzin gefällt. Das nach dem Zentrifugieren erhaltene Sediment wird in 0.1 % Zitrat gelöst und dieser Vorgang der Umfällung so oft wiederholt, bis eine Probe des erhaltenen Sediments in destilliertem Wasser aufgenommen, mit einem Fibrinogengehalt von 0.1% - 0.2% bei 37°C mit einem PTT Reagenz eine aPTT von nicht weniger als 200 sec. ergibt. Ebenso muß nach Zusatz von Thromboplastin und Taipan Viper Venom eine Gerinnungszeit erreicht werden, die nicht unter 300 sec. liegt.

Das letzte, zur weiteren Verarbeitung geeignete, wiederaufgelöste Präzipitat wird durch Diafiltration gegen eine 0.1%ige Zitratlösung von Glyzin weitgehend befreit und dabei auf 2% - 3% Proteingehalt konzentriert.

Das so gewonnene Fibrinogen enthält auch noch wesentliche Mengen Fibronektin. Falls erwünscht, kann Fibronektin durch Ausfallung bei -2° C bis - 3 °C mit 17%igem Glyzin abgetrennt werden. Dabei wird praktisch alles Fibrinogen gefällt und im Überstand befindet sich Fibronektin mit noch geringen Mengen Fibrinogen. Durch Sättigung mit Glyzin können beide Proteine gemeinsam ausgefällt und gewonnen werden. Allenfalls vorhandene, geringe Aktivitäten an Plasminogenaktivator oder Plasmin können durch Zusatz von geringen, niedermolekularen Inhibitoren, wie Epsilonaminokapronsäure oder Derivate in ihrer Wirkung aufgehoben werden.

Die Virusinaktivierung des hochgereinigten Fibrinogens oder des Fibrinogen-Fibronektin-Komplexes kann durch Versetzen des Plasmas der gelösten Cohn Fraktion-I oder des gelösten Kältepräzipitats mit Detergenzien und Netzmitteln erfolgen oder mit einem Hitzeimpuls- oder Laserlichtverfahren. Die virusinaktivierte Fibrinogenlösung kann als solche bei Kühlschranktemperatur bei 4° C bis 8° C gelagert, zur Lagerung tiefgefroren oder gefriergetrocknet werden.

### 2. Beispiel (vergleichend):

Herstellung einer Thrombinlösung mit thrombingenerierendem Potential.

Diese Thrombinlösung wird durch Vermischen zweier Lösungen zu gleichen Teilen, die aus zur Herstellung von Arzneimitteln für die Anwendung am Menschen geeignetem menschlichem Plasma gewonnen wurden, erhalten. Beide Lösungen sind pyrogenfrei, Caionenfrei und steril. Eine Lösung enthält virussicheres Thrombin in einer auszuwählenden Konzentration von 20 bis 2.500 Einheiten/ml, die andere enthält ein virussicheres Prothrombin-Gerinnungsfaktoren-Gemisch, das pro ml nach Ca-ionen-Zusatz mindestens 1.000 Thrombin-Einheiten generieren kann.

Diesem Gemisch wird ein hundertstel Volumsteil einer sterilen Lösung von 10% Polyäthylenglykol (pharmazeutische Reinheit) beigemischt und das Gemisch dann je nach gewünschter Dosierung in Durchstichfläschchen oder in Fertigspritzen verfüllt und tiefgefroren, allenfalls gefriergetrocknet, verschlossen, gelagert, etikettiert und verpackt.

### 3. Beispiel (vergleichend) :

Herstellung von für den Menschen geeigneten Arzneimitteln zur lokalen Anwendung aus fibrinhältigen und thrombinhältigen pharmazeutischen Zubereitungen.

800 ml einer pyrogenfreien flüssigen Wirkstoffzubereitung nach Beispiel 1, die aus zur Herstellung von Arzneimitteln für den Menschen geeignetem menschlichem Plasma gewonnen wurde und einen Fibrinogengehalt von mindestens 6 % aufweist, werden zur Auflösung einer gefriergetrockneten, sterilen, pyrogenfreien, virusinaktivierten, aus zur Herstellung von Arzneimitteln für den Menschen geeignetem menschlichem Plasma gewonnenen pharmazeutischen Zubereitung verwendet, die 1.200 arbiträre, auf menschliches Plasma bezogene Einheiten PAI-1 oder eines anderen Serpins, bzw. Serpingemisches, das keine kollagenase- oder elastasehemmende Wirkung aufweist und ein Transglutaminasezymogengehalt äquivalent zu mindestens 4.000 Einheiten Faktor XM enthält. Weiters enthält die Zubereitung 2 g CaCl₂.

Diese fibrinogenhältige pharmazeutische Zubereitung wird nun mit der im Beispiel 2 angegebenen thrombinhältigen Zubereitung nach deren Rekonstitution mit der entsprechenden Menge Wassers für Injektionszwecke vermischt, und zwar 4 Teile der fibrinogenhältigen Zubereitung mit 1 Teil der thrombinhältigen Zubereitung.

### 4. Beispiel (vergleichend) :

Herstellung einer allogenen, provisorischen, extrazellulären Matrix als Arzneimittel.

Eine bestimmte Menge des Gemisches nach Beispiel 3 wird in eine gewünschte sterilisierte Form eingegossen und 5 Stunden unter sterilen Bedingungen zwischen 35° C und 37° C mkubiert. Die sich in der Form gebildete sterile, pyrogenfreie, virussichere, für die Verwendung beim Menschen allogene, extrazelluläre Matrix wird in sterile, wasserdampfdichte Polyäthylenhüllen verpackt, verschweißt, und bei Kühlschranktemperatur 4 ° C - 8 ° C zur Freigabe nach den entsprechenden Qualitätskontrollen gelagert. Nach Etikettierung, Verpackung und Freigabe kann das Arzneimittel in den Verkehr gebracht werden.

### 5. Beispiel (vergleichend)

Herstellung einer allogenen, provisorischen, extrazellulären Matrix durch Vermischen dreier pharmazeutischer Zubereitungen, die als zusammengesetztes Arzneimittel angeboten werden.

Das Arzneimittel enthält drei pharmazeutischer Zubereitungen, die nach Vermischen eine allogene, provisorische, extrazelluläre Matrix ausbilden:
a. eine 6%ige Fibrinogenlösung nach Beispiel 3,
b. gefriergetrocknete Mischung von Transglutaminasezymogen, Serpin oder Serpingemisch, frei von kollagenase- und elastasehemmender Wirkung und Calciumchlorid in den in Beispiel 3 angegebenen Mengen.
c. eine gefriergetrocknete Thrombinlösung nach Beispiel 2.

Im Arzneimittel ist weiters Wasser für Injektionszwecke vorhanden, das benützt wird, um die thrombinhältige pharmazeutische Zubereitung gemäß Beispiel 2 aufzulösen, während die pharmazeutische Zubereitung, die das Transglutaminasezymogen und das Serpin enthält, in der 6%igen Fibrinogenlösung nach Beispiel 3 gelöst wird. Nach Vermischen der fibrinogenhältigen mit der thrombinhältigen Lösung wird vor der Gerinnung dieses Gemisches dieses entweder in eine gewünschte, sterile, pyrogenfreie Form eingebracht und nach Verfestigung aus der Form entfernt und lokal aufgebracht oder das noch flüssige Gemisch lokal auf die gewünschte Stelle bzw. die gewünschten Stellen aufgebracht, wobei sich erst dann lokal eine für den Menschen allogene, provisorische extrazelluläre Matrix ausbildet.

### 6. Beispiel (vergleichend) :

Biomechanisch verstärkte, allogene, provisorische extrazelluläre Matrix unter Verwendung von allogenem Kollagen.

In das Gemisch der pharmazeutischen Zubereitungen nach Beispiel 4 und 5 kann noch fibriläres, steriles, pyrogenfreies, virussicheres, menschliches Kollagen eingebracht werden, und zwar zwischen 5 - 100 mg/ml, oder das Gemisch wird auf ein schnell saugfähiges, schawnförrniges Kollagenfließ aufgebracht, wobei der Thrombingehalt des Gemisches so eingestellt ist, daß die Fibrinbildung erst nach Ende des Aufsaugungsvorganges eintritt. Ein solches Fließ kann unmittelbar nach Einsetzen des Gerinnungsvorganges verwendet werden oder nach Inkubation von 5 Stunden zwischen 35° C und 37° C, steril verpackt, zum Fertigarzneimittel weiter- verarbeitet werden.

## Patentansprüche

1. Verfahren zur Herstellung einer fibrinogenhältigen Lösung, die bei Kühlschranktemperatur oder Raumtemperatur lagerfähig ist, **dadurch gekennzeichnet, daß** die Fibrinogenlösung aus gentechnologisch hergestelltem Fibrinogen oder aus Plasma gewonnenem Fibrinogen durch Fraktionierung mit Glyzin bei Temperaturen unter 0°C hergestellt wird.

## Claims

1. A process for preparing a fibrinogen-containing solution which is storable at refrigerator temperature or room temperature, **characterized in that** the fibrinogen solution is prepared of fibrinogen produced by genetic engineering or of fibrinogen obtained from plasma by fractionation with glycine at temperatures of below 0°C.

## Revendications

1. Procédé destiné à la fabrication d'une solution contenant du fibrinogène, pouvant être stockée à la température d'un réfrigérateur ou à la température ambiante, **caractérisé en ce que** la solution de fibrinogène est fabriquée à partir de fibrinogène élaboré par génie génétique ou à partir de fibrinogène récupéré du plasma par un fractionnement à l'aide de glycine à des températures en dessous de 0 °C.
